# EUROPEAN PATENT APPLICATION

(11) **EP 2 952 095 A1**
(43) Date of publication of application: **09.12.2015**
(21) Application number: 15172729.4
(22) Date of filing: 25.10.2006
(51) Int. Cl.: A01N 43/16, A01N 55/02, A01N 47/30, A01N 47/12, A01N 43/90, A01N 43/80, A01N 43/78, A01N 43/40, A01N 41/10, A01N 35/08, A01N 31/16, A01N 31/08, C11D 10/06, A01P 1/00, A01N 59/16

(54) **ANTIMICROBIAL COMPOSITION AND METHOD**

(30) Priority: 25.10.2005 US 730142 P
(62) Divisional of application: 14154982.4
(71) Applicant: Dow Global Technologies LLC, Midland, MI 48674 (US)
(72) Inventor: GARTNER, Charles D., Midland, Michigan 48642 (US); ANNIS, Ioana, Mundelein, Illinois 60066 (US)
(74) Representative: Beck Greener

(57) **Abstract**

An antimicrobial composition containing a cationic polymer having limited antimicrobial activity (such as a hydrophobically-modified quaternary ammonium cellulose ether) and an antimicrobial compound (such as one or more compounds selected from the group consisting of sodium pyrithione and zinc pyrithione). The growth of microorganisms (such as *Pseudomonas aeruginosa*) can be inhibited by exposing the microorganism to such a composition.

## Description

### CROSS-REFERENCE TO RELATED APPLICATIONS

This application claims priority from provisional application serial number 60/730,142, filed October 25, 2005, which is incorporated herein by reference in its entirety.

### BACKGROUND OF THE INVENTION

The invention is in the field of antimicrobial compositions and more specifically the invention is in the field of antimicrobial compositions comprising a combination of compounds.

Microbial contamination of water-containing products must be considered and controlled to prevent product degradation, break down of processing equipment, and other serious economic and health hazards. The use of antimicrobial agents is vital for the prevention of microbial proliferation. The choice of antimicrobial agent is heavily application specific, taking into consideration various factors such as use conditions and performance expectations. The majority of antimicrobial agents are not equally efficient across the entire microorganism spectrum. To compensate for the gaps in performance, several practices are employed in the field. Examples of these are: use of excess antimicrobial agent; use of several antimicrobials in synergistic or additive combinations; use of performance enhancing agents, such as surfactants, chelants, salts; and the addition of antimicrobials at different stages in the process.

Cationic polymers are widely used in a large variety of applications. Specific examples involve use as thickeners, dispersion stabilizers, flocculants and binders, controlled release agents. The cationic cellulose ethers are extensively employed in the personal care and the household cleaning due to their ability to impart several desirable qualities to the final formulation, such as improved deposition, good filming characteristics, soft feel on hair and skin, and hair manageability. Most cationic polymers have little antimicrobial activity.

There remains a need for antimicrobial compositions which provide improved performance, increased safety and reduced cost.

### SUMMARY OF THE INVENTION

The invention addresses these needs by introducing high efficacy blends of antimicrobial compounds with cationic polymers. The use of cationic polymers in the invention presents dual benefits: (a) enhancement of the antimicrobial efficacy against organisms that the antimicrobial agents control at acceptable concentrations, and (b) broadening of the efficacy spectrum against organisms that the antimicrobial agents alone can control only at very high active concentrations, or not at all.

More specifically, the invention is an antimicrobial composition comprising: a cationic polymer having limited antimicrobial activity (such as a hydrophobically-modified quaternary ammonium cellulose ether); and an antimicrobial compound (such as one or more compounds selected from the group consisting of diiodomethyl-para-tolylsulfone, ortho-phenylphenol, sodium pyrithione, zinc pyrithione, 3-iodo-2-propynylbutylcarbamate, 2-methyl-4-isothiazolin-3-one, 1,2-benzisothiazolin-3-one, 2-n-octyl-4-isothiazolin-3-one, 1-(3-chloroallyl)-3,5,7-triaza-1-azoniaadamantane chloride, 2-(4-thiazolyl)-benzimidazole, β-bromo-β-nitrostyrene, 2,4,4'-trichloro-2-hydroxyphenyl ether, chloroxylenol, chlorocresol, para-tert-amylphenol, N-(4-chlorophenyl)-N'-(3,4-dichlorophenyl)-urea, and para-hydroxybenzoic acid esters). In another embodiment, the invention is a method of inhibiting the growth of a microorganism by the step of exposing the microorganism to such a composition.

### DETAILED DESCRIPTION

It is a feature of this invention to provide a family of antimicrobial compositions capable of controlling the growth of at least one microorganism, for example fungi, bacteria, yeast, algae, and mixtures thereof. The compositions of the present invention could behave as fast acting antimicrobial agents and/or prolonged activity, slower acting, antimicrobial agents. Methods of inhibiting or controlling the growth of at least one microorganism are also a feature of this invention.

Provided here are compositions and methods for controlling the growth of at least one microorganism by using at least one cationic polymer having limited antimicrobial activity and one or more antimicrobial agents such as diiodomethyl-para-tolylsulfone (DIMTS, Amical^{®}), ortho-phenylphenol (OPP), sodium pyrithione (NaPT), zinc pyrithione (ZPT), 3-iodo-2-propynylbutylcarbamate (IPBC), 2-methyl-4-isothiazolin-3-one (MIT), 1,2-benzisothiazolin-3-one (BIT), 2-n-octyl-4-isothiazolin-3-one (OIT), 1-(3-chloroallyl)-3,5,7-triaza-1-azoniaadamantane chloride (CTAC, Dowicil 200), 2-(4-thiazolyl)-benzimidazole (TBZ, thiabendazole), β-bromo-β-nitrostyrene (BNS), 2,4,4'-trichloro-2-hydroxyphenyl ether (Triclosan), chloroxylenol (PCMX), chlorocresol (PCMC), para-tert-amylphenol (PTAP), N-(4-chlorophenyl)-N'-(3,4-dichlorophenyl)-urea (Trichlocarban), para-hydroxybenzoic acid esters (parabens), and mixtures thereof. Preferred antimicrobial agents are DIMTS, OPP, NaPT, ZPT, IPBC, BIT, OIT, TBZ, BNS, 2,4,4'-trichloro-2-hydroxyphenyl ether, chloroxylenol, chlorocresol, PTAP, N-(4-chlorophenyl)-N'-(3,4-dichlorophenyl)-urea, and mixtures thereof.

The compositions provide superior antimicrobial control at a significantly lower concentration of antimicrobial agent. The compositions preferably contain the optimal amount of the cationic polymer to obtain desired antimicrobial efficacy at the lowest possible antimicrobial agent concentration.

In an embodiment of the invention, the antimicrobial agent is of limited water solubility. By "limited water solubility" is meant a solubility in water of 0.5 wt % or less, more preferably 0.25 wt % or less, and even more preferably, 0.1 wt % or less, at 25 °C. Examples of antimicrobial agents having limited water solubility include: diiodomethyl-para-tolylsulfone (DIMTS, Arnica^{®}), ortho-phenylphenol (OPP), zinc pyrithione (ZPT), 3-iodo-2-propynylbutylcarbamate (IPBC), 1,2-benzisothiazolin-3-one (BIT), 2-n-octyl-4-isothiazolin-3-one (OIT), 2-(4-thiazolyl)-benzimidazole (TBZ, thiabendazole), 2,4,4'-trichloro-2-hydroxyphenyl ether (Triclosan), chloroxylenol (PCMX), chlorocresol (PCMC), para-tert-amylphenol (PTAP), N-(4-chlorophenyl)-N'-(3,4-dichlorophenyl)-urea (Trichlocarban), para-hydroxybenzoic acid esters (parabens). The inventors have discovered that the efficacy of antimicrobial agents with limited water solubility is enhanced when formulated with a cationic polymer as described herein.

In a further embodiment, the following antimicrobial agents are excluded from the compositions and methods of the invention: bronopol; Ag (metal) and Ag(+) complexes; ionene; peracids; biguanides and bisbiguanides; and chlorhexidine and its salts.

The invention also provides methods for controlling or inhibiting the growth of at least one microorganism in or on a product, material or medium susceptible to microorganism proliferation. The methods include the step of adding to the product, material, or medium a composition of the present invention, in an effective amount to control microorganism growth. It should be understood by those skilled in the art that the choice of the cationic polymer, the choice of the antimicrobial agent(s), and the effective amount, will vary in accordance with the product, material, or medium to be treated. According to the invention the components can be added individually such that the final amount of each component present at the time of use is effective to control the growth of at least one microorganism at a lower concentration of the antimicrobial agent than would be required if no cationic polymer was present.

The compositions of the invention are useful to control the growth of at least one microorganism in various types of materials, products or media susceptible to microbial attack. They are also useful in controlling the growth of at least one organism on a product or surface treated with the formulation containing a composition of the invention. Nonlimiting examples include: personal care products, skin care products, toothpaste, household cleaners and disinfectants, dish washing products, hard surface disinfectants, toilet bowl cleaners and disinfectants, laundry detergents and softeners, dyes, adhesives, paints, mineral slurries, leather, textile, pulp and paper, wood, tanning liquor, polymer emulsions, metalworking fluids, lubricants, oil field drilling fluids and muds, process water, waste water, starch, proteinaceous materials, recreational water, paper coatings and sizing agents, agrochemicals and agricultural applications, petrochemicals. The identity and the most effective amounts of cationic polymer and antimicrobial agent(s) vary in accordance to the material, process or medium to be treated, and optimization can be readily achieved by those skilled in the art.

The compositions may further include surfactants, such as cationic, anionic, nonionic or amphoteric surfactants; additional antimicrobial agents; dispersion agents; salts; pH modifiers; UV absorbers; rheology modifiers; surface agents; chelants; dyes; fragrance; opacifiers, foam suppressors, etc.

The use concentration for the antimicrobial compound of the invention is preferably at least about 0.01 %, more preferably at least about 0.5 %, by weight based on the total weight of the formulation, and preferably no more than about 10 %, more preferably no more than about 5 %, even more preferably no more than about 3 % by weight, and even more preferably no more than about 1 % by weight.

The concentration of cationic polymer is preferably at least about 0.01 %, more preferably at least about 1 % by weight, and preferably no more than about 10 %, more preferably no more than about 5 % by weight.

The weight ratio of cationic polymer relative to antimicrobial agent is preferably no more than about 200:1, more preferably no more than about 100:1 and even more preferably no more than about 50:1. In further embodiments, the weight ratio of polymer relative to antimicrobial is no more than about 25:1, no more than about 10:1, no more than about 5:1, or no more than about 2:1. Preferably the ratio of cationic polymer relative to antimicrobial is at least about 1:1. In further embodiments, the ratio is at least about 2:1, at least about 5:1 or at least about 10:1.

As used herein, the terms "antimicrobial" and "inhibiting microbial growth" describe the killing of, as well as the inhibition of or control of, the growth of bacteria, yeasts, fungi, and algae. Enhancement of antimicrobial efficacy refers to increasing the rate of kill and/or decreasing the amount of necessary antimicrobial agent to achieve antimicrobial control. The term "limited antimicrobial activity" in reference to cationic polymers having limited antimicrobial activity means that such cationic polymers inhibit or decrease antimicrobial growth by no more than 2 log after 24 h contact and no more than 4 log after 7 days, at use-concentration of 0.5% to 5%. The term "antimicrobial compound" means a compound that inhibits or decreases antimicrobial growth by more than 2 log after 24 h contact and more than 4 log after 7 days, at use-concentration of 0.5% to 5%.

The compositions of the invention have applications in several markets both as preservatives and/or antimicrobial agents. These markets include: personal care, such as but not limited to, shampoos, conditioners, face and body lotions, or active antimicrobial ingredients in antifungal shampoo and conditioners, solid and liquid soaps, toothpaste, facial cleansing products, and anti-acne products, "waterless" antimicrobial rubs or wipes; household cleaning and disinfection applications, such as preservative and/or antimicrobial agent in dishwashing products and laundry detergents and softeners, hard surface disinfection, products for toilet bowls cleaning and disinfection, hard surface antimicrobial wipes; institutional and industrial disinfection; agrochemical applications; industrial water treatment including cooling water, process water, waste water, pulp and paper, oil field treatment, and drilling mud preservative; metal working systems; porta-potties cleaning and disinfection; as antimicrobial agents to prevent the contamination of plastic, cardboard, wood, paper, wallboard, grout, tape-joint compounds, adhesives and sealants; preservation of aqueous paints, adhesives, latex emulsions, and joint cements; wood preservation; preserving cutting oils; preservation systems for textiles and leather; as a component of antifouling paints to prevent adherence of fouling organisms; protecting paint films, especially exterior paints, from attack by fungi which occurs during weathering of the paint film; protecting processing equipment from slime deposits during manufacture of cane and beet sugar; preventing microorganism buildup and deposits in air washer or scrubber systems and in industrial fresh water supply systems; in swimming pools to prevent algae growth.

In a preferred embodiment, the compositions of the invention are useful in soap bars and liquid soap compositions. Conventional antibacterial soaps generally contain germicides or antibacterial agents. Thus, for example, soap bars and liquid soaps containing antimicrobials such as triclosan (i.e., 2,4,4'-trichloro-2'-hydroxy-diphenylether) and triclocarbanilide are known. However, the addition of antibacterial agents to soap to achieve antibacterial effectiveness can add cost to the soap because of the cost of the antibacterial agents themselves and the added costs of production of the soap. By providing the antimicrobial in the form of a composition according to the invention, the efficacy of the antimicrobial is significantly increased. Consequently, less antimicrobial is required and the cost for manufacturing the soap is reduced.

A soap formulation according to the invention preferably contains at least about 0.01 %, more preferably at least about 1 %, by weight of an antimicrobial compound (described above), and preferably no more than about 10 %, more preferably no more than about 5 %, and even more preferably no more than about 3 % by weight of the antimicrobial. The soap preferably contains at least about 0.01 %, more preferably at least about 1 %, by weight of an the cationic polymer (described above), and preferably no more than about 10 %, more preferably no more than about 5 % by weight. The weight ratio of cationic polymer relative to antimicrobial is preferably no more than about 200:1, more preferably no more than about 100:1 and even more preferably no more than about 50:1. In further embodiments, the ratio is no more than about 25:1, no more than about 10:1, no more than about 5:1, or no more than about 2:1. Preferably the weight ratio of cationic polymer to antimicrobial agent is at least about 1:1.

The soap compositions of this embodiment comprise one or more "fatty acid soaps," which, for purposes of describing this component of the soap of the present invention, has the meaning as normally understood in the art: monovalent salts of monocarboxylic fatty acids. The counterions of the salts generally include sodium, potassium, ammonium and alkanolammonium ions, but may include other suitable ions known in the art.

Typically, the fatty acid soap components in the soap formulation comprise salts of long chain fatty acids having chain lengths of the alkyl group of the fatty acid from about 8 carbon atoms to about 18 carbon atoms in length. In some embodiments, the chain length is preferably between about 8 and about 10 carbon atoms. In other embodiments, the chain length is preferably between about 12 and about 18 carbon atoms. The particular length of the alkyl chain(s) of the fatty acid soaps is selected for various reasons, including cleansing capability, lather capability, costs, and the like. It is known that soaps of shorter chain lengths are more water-soluble (i.e., less hydrophobic) and produce more lather compared to longer chain length soaps. Longer chain length soaps are often selected for cost reasons and to provide structure to the soap. Typically, a soap composition according to the invention comprises at least about 45 %, more preferably at least about 60 %, by weight of fatty acid soap, and less than about 95 %, more preferably less than about 90 %, by weight of the fatty acid soap. The soap bars and liquid soap formulations may also include optional adjuvant ingredients such as moisturizers, humectants, water, fillers, polymers, dyes, fragrances and the like to effect cleansing and/or conditioning for the skin of the user.

Preferred antimicrobial agents for the soap composition include: 2,4,4'-trichloro-2-hydroxyphenyl ether (Triclosan), chloroxylenol (PCMX), chlorocresol (PCMC), para-tert-amylphenol (PTAP), and N-(4-chlorophenyl)-N'-(3,4-dichlorophenyl)-urea (Trichlocarban). Preferred cationic polymers include cationic polysaccharides and hydrophobically-modified cationic polysaccharides.

The invention further comprises a method of controlling or inhibiting microbial growth by using a cationic polymer having limited antimicrobial activity together with one or more antimicrobial compounds such as diiodomethyl-para-tolylsulfone (DIMTS, Arnical^{®}), ortho-phenylphenol (OPP), sodium pyrithione (NaPT), zinc pyrithione (ZPT), 3-iodo-2-propynylbutylcarbamate (IPBC), 2-methyl-4-isothiazolin-3-one (MIT), 1,2-benzisothiazolin-3-one (BIT), 2-n-octyl-4-isothiazolin-3-one (OIT), 1-(3-chloroallyl)-3,5,7-triaza-1-azoniaadamantane chloride (CTAC, Dowicil 200), 2-(4-thiazolyl)-benzimidazole (TBZ, thiabendazole), β-bromo-β-nitrostyrene (BNS), 2,4,4'-trichloro-2-hydroxyphenyl ether (Triclosan), chloroxylenol (PCMX), chlorocresol (PCMC), para-tert-amylphenol (PTAP), N-(4-chlorophenyl)-N'-(3,4-dichlorophenyl)-urea (Trichlocarban), and para-hydroxybenzoic acid esters (parabens).

The term "cationic polymer" as used herein, refers to a polymer having as some, or all, of its side-chains either cationic groups or groups capable of being quaternized by adjusting the pH of the polymeric solution. Most often, these groups are quaternary ammonium moieties, however other cationic moieties are covered by this invention. As specified above, many of these cationic materials present limited or no antimicrobial activity when used alone, especially against Gram negative organism such as *Pseudomonas aeruginosa.* Specific cationic polymers useful in this composition, include but are not limited to: cationic polysaccharides, hydrophobically-modified cationic polysaccharides, cationic proteins, cationic polynucleotides, cationic glycoproteins, and cationic glycosaminoglycans. Preferably, the cationic polymer for use in the invention has a number average molecular weight of at least about 30,000, more preferably at least about 40,000, and even more preferably at least about 50,000.

One preferred class of cationic polymers is cationic polysaccharides, and especially, cationic cellulose derivatives which are classified under the CTFA (Cosmetic, Toiletry, and Fragrance Association) designation Polyquaternium-10 and are commercially available under the trade name UCARE™ polymers (e.g., JR30M) from Amerchol Corp., Edison, N.J., USA. These polymers contain quaternized ammonium groups grafted to a cellulosic polymer chain.

Even more preferred cationic polymers are classified under the CTFA designation Polyquaternium-67 and are commercially available under the trade name SoftCat™ SL polymers (e.g., SL 100) from Amerchol Corp., Edison, N.J., USA. These polymers contain hydrophobically-modified quaternized ammonium groups grafted to a cellulosic polymer chain.

Other cationic polymers useful in the invention include, without limitation thereto, quaternary ammonium derivatives of guar gum, such as hydroxypropyl guar triammonium chloride; quaternized chitosan or quaternized chitosan derivatives; quaternary ammonium salts of acrylic and methacrylic copolymers, such as polymethacrylamidopropyl trimonium chloride, acrylamidopropyl trimonium chloride - acrylamide copolymers; quaternary ammonium salts of copolymers of vinylpyrrolidone and acrylates or methacrylates; quaternary ammonium salts of copolymers of vinyl caprolactam, polyvinylpyrrolidinone and acrylates or methacrylates; quaternized polyaminoacids; polyaminoacids that could be quaternized under specific pH conditions, such as polylysine; polyamines; hydrophobically-modified polyamines, such as benzylated, ethoxylated or propoxylated polyamines; polyvinylamines; other nitrogen-containing polymers such as biguanides.

The specific cationic polymer used is preferably selected based on compatibility with the application. Selection criteria may include solubility, lack of reactivity with other system components, and viscosity. Moreover, it is important to select cationic polymers that have optimal interaction with the antimicrobial agent of choice. Those skilled in the art are able to select suitable cationic polymer candidates for the specific application and to optimize the use amounts and conditions.

The compositions of the invention may be prepared as liquids, dispersions or emulsions, solids, or in aerosol form. The compositions of the invention may be applied by spraying or brushing on the material or product, by dipping the material or product in a suitable formulation of the composition. The compositions of the invention can be added into a material, product or medium by pouring or metering-in with a suitable device.

In the invention, the use of the cationic polymers having limited antimicrobial activity together with an antimicrobial agent selected from the list given above, provides antimicrobial control at active concentrations significantly lower than those required when the antimicrobial is used alone. Depending on the structure and mode of action of the antimicrobial agent different types of activity enhancement was noted. With regard to strong fungicides, such as DIMTS and ZPT, which present limited antibacterial efficacy, use of the cationic polymer significantly boosts and accelerates the antibacterial efficacy. For example, in the case of DIMTS, 2000 ppm DIMTS provides 1 log reduction in *P. aeruginosa* count after 24 h and 48 h contact, and only 2 log reduction after 7 days of contact. Use of 414 ppm DIMTS in conjunction with 0.5% SoftCat SL100 cationic polymer results in 1 log reduction after 24 h, 3.5 log reduction after 48 h, and 4 log reduction after 7 days, representing a greater than 5-fold increase in activity using the invention. In the case of ZPT, 800 ppm active is needed to give over 4 log reduction in *P. aeruginosa* population after 7 days of contact. This concentration gives 2 log reduction after 48 h, and no control before 48 h. Use of 46.8 ppm active in conjunction with 0.5% SoftCat SL 100 cationic polymer provides 1 log reduction after 24 h, 3.5 log reduction after 48 h, and 4 log reduction after 7 days, representing a 17-fold increase in activity using the invention.

A similar pattern is noted for slow acting preservatives, such as CTAC. When used alone, 400 ppm active provides 6 log reduction in *S. aureus* count after 7 days of contact. Use of 79 ppm active together with 0.25% UCARE JR 30M cationic polymer achieves 1 log reduction after 24 h contact, 1.5 log reduction after 48 h and 4 log reduction at 7 days. Thus, the activity of the CTAC is both enhanced and accelerated by use of the cationic polymer of the invention. And, when 400 ppm active are used in conjunction with the polymer, a 4 log reduction is obtained after only 24 h of contact.

In a specific embodiment of this invention, use of the cationic polymers together with Triclosan significantly improve the efficiency of Triclosan against *Pseudomonas aeruginosa.* Thus, 800 ppm of Triclosan achieved 3 log reduction in *P. aeruginosa* only after greater than 48 hours contact time. Use of either SoftCat™ SL 100 or UCARE™ JR30M significantly accelerated and increased the rate of kill. Detailed examples are given in Table 3 below. Specifically 355.6 ppm Triclosan in the presence of 0.25 % SoftCat™ SL 100 achieved 3 log reduction at time zero. When the concentration of Triclosan was further reduced to 105.3 ppm the log reduction decreased only by half of log. Similarly use of 355.6 ppm of Triclosan in conjunction with 0.25% UCARE™ JR 30M also results in 3 log reduction at time zero. After 7 days of contact time, all the listed polymer-Triclosan combinations achieve greater than 3-log reduction. By comparison, a more moderate increase in antimicrobial efficacy was noted with Ecopol 13 (cationic guar gum).

For fast acting biocides, such as OPP, the use of the cationic polymer is antagonistic at the time zero point, however, synergy is noted at the later time-points. It is also recognized that the efficacy of the biocide may be prolonged as the cationic polymer might function as a control release agent. It should also be noted that the choice of cationic polymer is usually important for optimal results.

Without wishing to be bound by theory, we hypothesize that the significant increase in efficacy when using a cationic polymer having limited antimicrobial activity in conjunction with the antimicrobial agent is due to the cationic groups facilitating delivery and interaction of the active agent with the microbial surface. In the case of hydrophobically-modified cationic polymers, such as the SoftCat polymers, the hydrophobic moieties could also increase the homogeneity of the system by better dispersing the active in solution. For the very early time-points, the increase in viscosity of the total system due to the use of the cationic polymer might be responsible for the slower biocidal action.

We recognize that in a final formulation use of such cationic polymers may also have a positive effect on other characteristics, such as: the deposition and retention of active ingredients, reduced leaching of the active from a substrate, improved filming characteristics, reduced drag and improved flow dynamics, prolonged or control release of certain actives. We also recognize that a final formulation comprising among other ingredients the cationic polymer and one of the antimicrobial agents cited herein, might also contain additional antimicrobial agents for improved preservation or synergy.

### EXAMPLES

Cationic polymers having limited antimicrobial activity used in the following examples include SoftCat™ SL 100 (cellulose ether with a [2-hydroxy-3-(trimethylammonio)-propyl]-w-hydroxypoly(oxy-1,2-ethanediyl) chloride); UCARE™ JR 30M (cationic cellulose ether) obtained from Amerchol Corp., Edison, NJ; and Ecopol 13 (guar gum, hydroxypropyl-trimonium chloride) from Economy Polymers and Chemicals, Houston, TX. Antimicrobials used in the following examples include OPP, CTAC, and DIMTS from The Dow Chemical Company, Midland, MI; ZPT from Olin Chemicals, Norwalk, CT; while the others were obtained from Aldrich Chemicals, Milwaukee, WI.

The cationic polymers are pH adjusted in the range of 6-7.5. It should be noted that the Ecopol polymers require an acidic environment for complete dissolution. Once the polymer is dissolved, NaOH can be used to bring the pH to the desired range.

The efficacy of the antimicrobial combinations against *Staphilococcus aureus* ATCC # 6538 and *Pseudomonas aeruginosa* ATCC #10145 is measured. Testing is done in 96-well 1.5 ml polypropylene assay blocks using 96-well polypropylene sealing mats for covering the samples during incubation. The individual wells in the 96-well assay block are identified as follows: 12 columns numbered 1-12 across the top row of wells on the block, and 8 rows lettered A-H on the first column of the block. Columns 1 and 2 are reserved to test the activity of the biocide in buffer (pH 7.2) in the absence of the cationic polymer. Columns 11 and 12 are always reserved as control, to verify growth of the microorganism in the absence of the biocide. The preparation of the assay blocks involves the following steps in chronological order:
1. Preparation of the biocide solution in an appropriate solvent at a concentration equal to 10 times the final concentration desired in row A;
2. Addition of predetermined amount of the stock biocide solution to each well in row A;
3. Addition of a predetermined amount of the solvent to rows B-H and wells 11A and 12A;
4. Serial dilution of the biocide, by transferring a predetermined amount of the solution from row A to row B, mixing, and repeating the transfer down all the rows. After the mixing step in row H, the transferring amount is taken out and disposed of;
5. Inoculation of the buffer and polymer solutions with the test organism. For bacteria, a 24-hour culture is used. Following the incubation period, the organism concentration in the medium is determined by reading the OD at 620 nm. This culture is used to inoculate the buffer and the polymer solution to a bacterial concentration of 1E6 cfu/ml. Fungal challenges are performed with fungal spores isolated from inoculated SDA slants. The spores are collected by swiping the slant with a sterile cotton swab and re-suspended in tryptic soy broth. Tween 80 is added to aid in spore separation and the suspension was homogenized. The spore concentration is obtained by use of a hemacytometer. This suspension is used to inoculate the buffer and the polymeric solutions to a spore concentration of 3-5E6 cfu/ml;
6. Addition of the inoculated buffer or polymer solution to each well.
7. Thorough mixing of the blends in each well;
8. Sampling for the time 0, 24h, 48h and 7 days reading is accomplished using a 96-well 300 µl polystyrene microplate. Tryptic Soy broth (TSB) is used for the bacterial studies and Malt (YM) broth buffered at pH 7.4 for fungal studies;
9. The microplates are incubated at 35 °C for bacteria and 30 °C for fungus, until the control samples show adequate growth; and
10. Data is recorded as the log reduction in microorganism concentration relative to the inoculum.

**Table 1. Efficacy against Pseudomonas aeruginosa.**

| Biocidal blend | Active Conc. (ppm) | Log kilt t₀ | Log kill 24 h | Log kill 48 h | Log kill 7 days |
|---|---|---|---|---|---|
| 0.5 % Ecopol 13 | 0 | 0 | 0 | 0 | -1 |
| 0.5% JR30M | 0 | 0 | 0 | 0 | 0.5 |
| 0.5% SL100 | 0 | 0 | 0.5 | 0.5 | 0 |
| | | | | | |
| ZPT | 800 | 0 | 0 | 2 | 6 |
| | 533.3 | 0 | 0 | 1.5 | 3 |
| | 46.8 | 0 | 0 | 0 | 2 |
| ZPT- 0.5% SL100 | 800 | 1 | 1 | 4 | 6 |
| | 533.3 | 0.5 | 1 | 4 | 6 |
| | 46.8 | 0.5 | 1 | 3.5 | 4 |
| | | | | | |
| DIMTS | 2000 | 0 | 1 | 1 | 2 |
| | 414 | 0 | 0 | 1 | 2 |
| DIMTS - 0.5% SL100 | 2000 | 0 | 3 | 4 | 4 |
| | 414 | 0 | 1 | 3.5 | 4 |
| | | | | | |
| OPP | 222.2 | 4 | 4.5 | 3.5 | 4 |
| | 182.2 | 1.5 | 1.5 | 2.5 | 2.5 |
| | 130.2 | 0 | 0 | 1 | 1.5 |
| OPP - 0.5% SL100 | 222.2 | 2 | 6 | 6 | 4 |
| OPP - 0.25% JR30M | 182.2 | 0.5 | 1.5 | 4.5 | 4 |
| OPP - 0.5% Ecopol 13 | 130.2 | 0.5 | 5 | 3 | 1 |
| | | | | | |
| CTAC | 800 | 0 | 2.5 | 5 | 6 |
| | 355.6 | 0 | 1 | 4 | 6 |
| | 158 | 0 | 0 | 0.5 | 5 |
| | 46.8 | 0 | 0 | 0 | 0 |
| CTAC - 0.5% SL100 | 355.6 | 0.5 | 4 | 4 | 4 |
| CTAC - 1% JR30M | 355.6 | 0 | 5 | 5 | 6 |
| | 158 | 0 | 4 | 4.5 | 6 |
| | 46.8 | 0 | 1 | 2.5 | 5 |

**Table 2. Efficacy against Staphilococcus aureus.**

| Biocidal blend | Active Conc. (ppm) | Log kill t₀ | Log kill 24 h | Log kill 48 h | Log kill 7 days |
|---|---|---|---|---|---|
| 0.25 % Ecopol 13 | 0 | 0 | 0 | 0 | 0 |
| 0.5% JR30M | 0 | 0 | 0 | 0 | 0.5 |
| 0.5% SL100 | 0 | 0 | 1.5 | 2 | 3.5 |
| | | | | | |
| ZPT | 100 | 1 | 0.5 | 1.5 | 2 |
| | 29.6 | 0 | 0 | 1 | 1 |
| | 5.9 | 0 | 0 | 1 | 1 |
| ZPT - 0.5% SL100 | 100 | 1 | 6 | 6 | 6 |
| | 29.6 | 1 | 6 | 6 | 6 |
| | 5.9 | 0 | 6 | 6 | 6 |
| | | | | | |
| DIMTS | 2000 | 1 | 1 | 1 | 6 |
| | 1428.6 | 0.5 | 0 | 0 | 2 |
| | 265.6 | 0 | 0 | 0 | 0 |
| | 189.7 | 0 | 0 | 0 | 0 |
| DIMTS - 0.5% JR30M | 2000 | 2 | 3 | 3.5 | 6 |
| | 265.6 | 0 | 2 | 3.5 | 5 |
| | | | | | |
| DIMTS - 0.5% SL100 | 2000 | 0.5 | 0.5 | 6 | 6 |
| | 318.7 | 0.5 | 0.5 | 5 | 6 |
| | | | | | |
| DIMTS - 0.5% Ecopol 13 | 2000 | 2 | 2.5 | 6 | 6 |
| | 189.7 | 0 | 0 | 1.5 | 6 |
| | | | | | |
| OPP | 418.4 | 6 | 6 | 6 | 4 |
| | 278.9 | 0.5 | 3.5 | 3 | 4 |
| | 182.2 | 1 | 6 | 6 | 6 |
| | 124 | 0 | 2 | 2 | 6 |
| | | | | | |
| OPP - 0.5% SL100 | 418.4 | 3 | 6 | 6 | 6 |
| | 124 | 0 | 3 | 5 | 6 |
| | | | | | |
| OPP - 0.5% JR 30M | 418.4 | 6 | 6 | 5 | 6 |
| | 182.2 | 4.5 | 6 | 5 | 5 |
| | | | | | |
| OPP -0.25% Ecopol 13 | 418.4 | 6 | 6 | 5 | 6 |
| | 182.2 | 5 | 6 | 5 | 3.5 |
| | | | | | |
| CTAC | 400 | 0 | 0 | 0 | 6 |
| | 177.8 | 0 | 0 | 0 | 4 |
| | 79 | 0 | 0 | 0 | 1 |
| | 52.7 | 0 | 0 | 0 | 1 |
| CTAC - 0.25 SL100 | 400 | 0 | 2 | 6 | 6 |
| | 52.7 | 0 | 0 | 0 | 6 |
| CTAC - 0.25% JR 30M | 400 | 0 | 4 | 6 | 6 |
| | 79 | 0 | 1 | 1.5 | 4 |
| CTAC - 0.25 % Ecopol 13 | 400 | 0.5 | 6 | 6 | 0 |
| | 79 | 1 | 4 | 4 | 0 |

**Table 3. Efficacy against Pseudomonas aeruginosa.**

| Biocidal blend | Triclosan Conc. (ppm) | Log kill t₀ | Log kill 24 h | Log kill 48 h | Log kill 7 days |
|---|---|---|---|---|---|
| 0.5% JR30M | 0 | 0 | 0 | 0 | 0.5 |
| 0.5% SL100 | 0 | 0 | 0.5 | 0.5 | 0 |
| Triclosan | 800 | 1 | 1 | 2.5 | 4 |
| | 355.6 | 1 | 1 | 2 | 2 |
| | 105.3 | 0 | 1 | 1 | 2 |
| | | | | | |
| Triclosan - 0.5 % SL100 | 800 | 3 | 4 | 5 | 6 |
| | 355.6 | 3 | 4 | 5 | 5.5 |
| | 105.3 | 2 | 3 | 4.5 | 4.5 |
| Triclosan - 0.25 % SL100 | 800 | 3 | 4 | 5 | 5.5 |
| | 355.6 | 3 | 5 | 5 | 6 |
| | 105.3 | 2.5 | 3 | 5 | 5.5 |
| Triclosan - 0.5 % JR 30M | 800 | 1.5 | 3 | 4.5 | 4.5 |
| | 158 | 1.5 | 4 | 4 | 4.5 |
| | 105.3 | 1.5 | 3.5 | 3.5 | 4 |
| Triclosan - 0.25 % JR 30M | 800 | 2.5 | 4 | 4 | 4.5 |
| | 355.6 | 3 | 4 | 4.5 | 5 |
| Triclosan - 0.125 % JR 30M | 800 | 3 | 4 | 5.5 | 5 |
| | 105.3 | 2 | 4 | 4 | 5 |
| Triclosan - Ecopol 13 0.125% | 800 | 1.5 | 2 | 3.5 | 5 |
| | 533 | 0 | 1.5 | 3 | 3 |

Preferred embodiments of the invention include the following:
1. An antimicrobial composition, comprising: a cationic polymer having limited antimicrobial activity; and an antimicrobial compound.
2. The composition of embodiment 1, wherein the antimicrobial compound comprises one or more compounds selected from the group consisting of diiodomethyl-para-tolylsulfone, ortho-phenylphenol, sodium pyrithione, zinc pyrithione, 3-iodo-2-propynylbutylcarbamate, 2-methyl-4-isothiazolin-3-one, 1,2-benzisothiazolin-3-one, 2-n-octyl-4-isothiazolin-3-one, 1-(3-chloroallyl)-3,5,7-triaza-1-azoniaadamantane chloride, 2-(4-thiazolyl)-benzimidazole, β-bromo-β-nitrostyrene, 2,4,4'-trichloro-2-hydroxyphenyl ether, chloroxylenol, chlorocresol, para-tert-amylphenol, N-(4-chlorophenyl)-N'-(3,4-dichlorophenyl)-urea, and para-hydroxybenzoic acid esters.
3. The composition of embodiment 1, wherein the antimicrobial compound has a solubility in water of about 0.5 weight percent or less.
4. The composition of embodiment 1, wherein the cationic polymer is a cationic polysaccharide.
5. The composition of embodiment 1, wherein the cationic polymer is a hydrophobically-modified cationic polysaccharide.
6. The composition of embodiment 5, wherein the hydrophobically-modified cationic polysaccharide is a hydrophobically-modified cationic cellulose ether.
7. The composition according to embodiment 1, wherein the antimicrobial compound comprises one or more compounds selected from the group consisting of DIMTS, OPP, NaPT, ZPT, IPBC, BIT, OIT, TBZ, BNS, 2,4,4'-trichloro-2-hydroxyphenyl ether, chloroxylenol, chlorocresol, PTAP, and N-(4-chlorophenyl)-N'-(3,4-dichlorophenyl)-urea.
8. The composition of any of embodiments 1-7, further comprising one or more of a surfactant, a pH modifiers; a salts; a rheology modifier; a dispersion stabilizers; a chelant; a fragrance; a pigments; a dye, and a UV-active compound.
9. A method of inhibiting the growth of a microorganism by the step of exposing the microorganism to the composition of any of embodiments 1-8.
10. The method of embodiment 9, wherein the microorganism is *Pseudomonas aeruginosa.*
11. A bactericidal soap composition comprising:
   a fatty acid soap; and
   an antimicrobial composition according to any of embodiments 1-8.
12. The bactericidal soap composition of embodiment 11 comprising between about 45 weight percent and about 95 weight percent of the fatty acid soap.
13. The bactericidal soap composition of embodiment 11 wherein the weight ratio of cationic polymer to antimicrobial compound is between about 200:1 and 1:1.
14. The bactericidal soap composition of embodiment 11 wherein the antimicrobial compound is 2,4,4'-trichloro-2-hydroxyphenyl ether, chloroxylenol, chlorocresol, para-tert-amylphenol, N-(4-chlorophenyl)-N'-(3,4-dichlorophenyl)-urea, or mixtures thereof.
15. The bactericidal soap composition of embodiment 11 wherein the cationic polymer is a cationic polysaccharide.
16. The bactericidal soap composition of embodiment 11 wherein the cationic polymer comprises a hydrophobically-modified cationic polysaccharide.

### CONCLUSION

While the invention has been described above according to its preferred embodiments, it can be modified within the spirit and scope of this disclosure. This application is therefore intended to cover any variations, uses, or adaptations of the invention using the general principles disclosed herein. Further, the application is intended to cover such departures from the present disclosure as come within the known or customary practice in the art to which this invention pertains and which fall within the limits of the following claims.

## Claims

1. An antimicrobial composition, comprising: a cationic polymer having limited antimicrobial activity; and an antimicrobial compound, wherein the antimicrobial compound comprises one or more compounds selected from the group consisting of sodium pyrithione and zinc pyrithione.

2. The composition of Claim 1, wherein the antimicrobial compound has a solubility in water of about 0.5 weight percent or less.

3. The composition of Claim 1, wherein the cationic polymer is a cationic polysaccharide.

4. The composition of Claim 1, wherein the cationic polymer is a hydrophobically-modified cationic polysaccharide.

5. The composition of Claim 4, wherein the hydrophobically-modified cationic polysaccharide is a hydrophobically-modified cationic cellulose ether.

6. The composition of any of Claims 1-5, further comprising one or more of a surfactant, a pH modifiers; a salts; a rheology modifier; a dispersion stabilizers; a chelant; a fragrance; a pigments; a dye, and a UV-active compound.

7. A method of inhibiting the growth of a microorganism by the step of exposing the microorganism to the composition of any of Claims 1-6.

8. The method of Claim 7, wherein the microorganism is *Pseudomonas aeruginosa.*

9. A bactericidal soap composition comprising:
a fatty acid soap; and
an antimicrobial composition according to any of claims 1-6.

10. The bactericidal soap composition of claim 9 comprising between about 45 weight percent and about 95 weight percent of the fatty acid soap.

11. The bactericidal soap composition of claim 9 wherein the weight ratio of cationic polymer to antimicrobial compound is between about 200:1 and 1:1.

12. The bactericidal soap composition of claim 9 wherein the cationic polymer is a cationic polysaccharide or a hydrophobically-modified cationic polysaccharide.
